# EUROPÄISCHE PATENTSCHRIFT

(11) **EP 4 498 905 B1**
(45) Veröffentlichungstag und Bekanntmachung des Hinweises auf die Patenterteilung: **25.03.2026**
(21) Anmeldenummer: 23729753.6
(22) Anmeldetag: 31.05.2023
(51) Int. Cl.: A61B 5/022, A61B 5/00, G01L 1/16, G01L 5/00

(54) **VORRICHTUNG ZUR BLUTDRUCKMESSUNG**
DEVICE FOR BLOOD PRESSURE MEASUREMENT
DISPOSITIF DE MESURE DE LA PRESSION ARTÉRIELLE

(30) Priorität: 01.06.2022 DE 102022113794
(43) Veröffentlichungstag der Anmeldung: 05.02.2025
(73) Patentinhaber: iNDTact GmbH, 97076 Würzburg (DE)
(72) Erfinder: PETRICEVIC, Raino, 97074 Würzburg (DE); LAUNER, Clemens, 97204 Höchberg (DE)
(74) Vertreter: Dr. Gassner & Partner mbB
(86) Internationale Anmeldenummer: PCT/EP2023/064528
(87) Internationale Veröffentlichungsnummer: WO 2023/232856

(56) Entgegenhaltungen:
- WO-A2-00/17615
- US-A1- 2007 287 923
- US-A1- 2017 055 854

## Beschreibung

Die Erfindung betrifft eine Vorrichtung zur Blutdruckmessung.

Ein Blutdruckmessgerät, das auch als Sphygmomanometer bezeichnet wird, ist ein Messgerät, mit dem äußerlich, am Oberarm oder am Handgelenk, der arterielle Druck eines Patienten gemessen werden kann. Die nach unterschiedlichen Methoden arbeitenden Messgeräte zeigen den oberen (systolischen) und den unteren (diastolischen) arteriellen Druck mit unterschiedlicher Genauigkeit an.

Die handelsüblichen Messgeräte messen den arteriellen Druck an der Innenseite des Handgelenkes und sind einfach zu bedienen. Messgerät und Manschette bilden eine Einheit. Das Gerät wird auf der Innenseite des Handgelenkes, an der Pulsader, angelegt und mit der Manschette befestigt. Nach dem Start der Messung wird die Manschette durch eine elektrische Pumpe so lange auf einen initialen Messdruck aufgepumpt, bis kein Blut mehr durch die Arterie fließen kann. Durch ein elektrisch gesteuertes Ventil wird der Druck in der Manschette schrittweise reduziert. Sensoren erfassen den aktuellen Druck und die sich ändernden Blutflussgeräusche. Durch Mustererkennung registriert das Gerät die Punkte des systolischen und diastolischen arteriellen Blutdrucks. Daneben können weitere Kennzahlen wie z. B. Pulsfrequenz, Herzrhythmusstörungen erfasst und ein Gesamtbewertungsstatus ermittelt werden.

Moderne Oberarm-Messgeräte zeigen die Werte auf einem integrierten Bildschirm an. Die Manschette kann vom Messgerät getrennt werden, um unterschiedliche Manschettengrößen zu ermöglichen. Sie sind weniger anwenderfreundlich als Messgeräte mit Messung an der Innenseite des Handgelenkes und teurer. Für sie spricht aber die Genauigkeit der Messergebnisse.

Bei der indirekten arteriellen Druckmessung, häufig mit RR (nach Riva-Rocci) oder seltener durch "NIBP" ("non-invasive blood pressure") abgekürzt, wird der arterielle Druck mit Hilfe eines Blutdruckmessgerätes an einer Extremität, meist am Arm, gemessen.

Bei der auskultatorischen Messung wird eine Druckmanschette geeigneter Breite am Oberarm bis oberhalb des erwarteten arteriellen Druckes aufgeblasen. Beim langsamen Ablassen kann man das Auftreten und danach wieder das Verschwinden eines Korotkow-Geräusches mit Hilfe eines Stethoskops über der Arterie des Armes hören (auskultieren). Der Druck, der bei erstmaliger akustischer Wahrnehmbarkeit des Geräusches auf der Skala des Messgerätes abgelesen werden kann, entspricht dem oberen, systolischen arteriellen Druckwert; d. h., dass der systolische Druck in diesem Moment größer ist als der Druck der Manschette. Der Druck wird mit geeigneter Geschwindigkeit weiter abgelassen. Unterschreitet der Manschettendruck den minimalen arteriellen Druckwert, verschwindet das Geräusch. Dieser Wert wird als diastolischer Druck bezeichnet und als unterer Wert notiert. Die auskultatorische Messung ist das Standardverfahren der nichtinvasiven Messverfahren.

Auch bei der palpatorischen Messung wird eine Druckmanschette am Oberarm angelegt. Beim Ablassen des Druckes wird der Puls an der Arteria radialis getastet. Der Druck, der beim erstmals getasteten Puls auf der Skala des Messgerätes abgelesen werden kann, entspricht ungefähr dem oberen, systolischen arteriellen Druckwert. Der diastolische Wert kann auf diese Weise nicht ermittelt werden. Das Verfahren bietet sich für z. B. laute Umgebungen, insbesondere im Rettungsdienst, an. Noch ungenauer ist die palpatorische Messung ohne Druckmanschette, bei der mit zwei Fingern die Speichenarterie getastet wird, mit dem herznäheren Fingern das Blutgefäß so weit komprimiert wird, bis mit dem herzferneren Finger kein Puls mehr zu spüren ist. Die dabei vom herznäheren Finger ausgeübte Kraft ist ein ungefährer Anhaltspunkt für den Blutdruck.

Die oszillatorische Messung wird im Prinzip wie die beiden anderen Verfahren durchgeführt, der obere und untere Wert wird anhand des Amplitudenverlaufs eines pulssynchronen Zeigerausschlags am Messgerät abgeschätzt, das die Übertragung von Schwingungen der Gefäßwand auf die Druckmanschette darstellt. Bei der manuellen Messung lassen sich mit dieser Methode nur ungenaue Ergebnisse erzielen. Dieses Messverfahren wird jedoch recht zuverlässig von Messautomaten zur kontinuierlichen Überwachung, z. B. postoperativ im Aufwachraum, eingesetzt. Diese messen als Alternative zur kontinuierlichen invasiven Druckmessung den arteriellen Druck des Patienten im Intervall von wenigen Minuten. Das oszillatorische Messverfahren findet auch Anwendung in den mittlerweile weit verbreiteten Handgelenkmessgeräten.

Auf dem gleichen Prinzip beruht auch die Langzeitblutdruckmessung (ABDM). Dabei trägt der Patient permanent (in der Regel über einen ganzen Tag) eine Blutdruckmanschette, die sich in festgelegten Intervallen automatisch aufpumpt und misst, sowie ein Aufzeichnungsgerät. Diese Methode gilt als Goldstandard für die Erkennung und Beurteilung des Schweregrades einer arteriellen Hypertonie.

Auch eine Messung mittels einer Pulswellenanalyse ist möglich. Dabei werden optische Signale wie die Pulsformung der Arterien interpretiert und der Blutdruck aus diesen Daten geschätzt. Der Vorteil der Methode ist, dass dauerhaft sowie nichtinvasiv gemessen werden kann und dafür lediglich ein Armband ohne Druckmanschette nötig ist.

Die folgenden Druckschriften sind Beispiele für den relevanten Stand der Technik und betreffen Vorrichtungen zur Blutdruckmessung, deren Komponenten und zugehörige Messverfahren: DE 3004011 A1, DE 3632592C2, DE 4439253 A1, DE 10214220 A1, EP 0165505 A1, EP 0334652 B1, EP 0467 853 A1, WO 2005/046466 A1, WO 2009/141171 A2, EP 0744155 A1, US 5 025 793, US 2012/0238887A1, US 2013/0226015 A1, US 2019/0320980 A1, US2019/0374116 A1, WO2017/183106 A1, WO 2018/231711 A1, WO 2019/209679 A1, WO 2020/112555 A1, WO 2021/110597 A1.

Weitere einschlägige Vorrichtungen sind aus den Dokumenten US 2007/287923 A1 und US 2017/055854 A1 bekannt.

Diese herkömmlichen Vorrichtungen und Verfahren weisen den Nachteil auf, dass das Aufpumpen der Manschette als sehr unangenehm bis schmerzhaft empfunden wird. Vor allem bei älteren Personen können dadurch Hautläsionen entstehen. Insbesondere bei dünner Haut, Entzündungen und durch wiederholte Messungen bei hoher Frequenz sind diese Messverfahren unangenehm und können großflächige Druckstellen erzeugen.

Zwar schlägt die WO 2009/141171 A2 eine drucklose Blutdruckmessung vor, allerdings benötigt der dort offenbarte Sensor eine zusätzliche mit Luftdruck zu beaufschlagende Manschette zur Kalibrierung. Diese Kalibrierung muss in der Regel bei jedem Anlegen der Manschette wiederholt werden. Eine derartige Blutdruckmessung ist daher umständlich.

Es gibt demnach keine einfachen und vor allem kompakten Systeme, die gänzlich ohne eine solche unangenehme Druckbeaufschlagung mittels Luftkissen auskommen.

Der Erfindung liegt daher die Aufgabe zugrunde, eine Vorrichtung zur Blutdruckmessung anzugeben, die einfach aufgebaut ist und kein Luftkissen zur Druckbeaufschlagung aufweist.

Zur Lösung dieser Aufgabe ist eine Vorrichtung zur Blutdruckmessung mit den Merkmalen des Anspruchs 1 vorgesehen.

Die erfindungsgemäße Vorrichtung zur Blutdruckmessung umfasst einen Träger, einen an dem Träger angeordneten Biegesensor, der zum Erfassen einer Biegung des Trägers ausgebildet ist, zwei Schenkel, die an entgegengesetzten Seiten des Trägers abgewinkelt zu dem Träger angeordnet sind, und eine Auswerteeinheit, die zum Ermitteln eines Blutdruckwerts anhand von Sensorsignalen des Biegesensors ausgebildet ist.

Die Erfindung beruht auf der Idee, dass der Biegesensor durch die arterielle und venöse Pulsation des Blutdrucks eine Biegung erfährt, sodass die dabei erzeugten Sensorsignale ausgewertet werden können, um den Blutdruck zu ermitteln. Die auf den Biegesensor ausgeübte Biegung erzeugt eine Zugspannung, wodurch die Sensorsignale hervorgerufen werden. Die erfindungsgemäße Vorrichtung zur Blutdruckmessung ermöglicht eine besonders sensible Messung des Blutdrucks bzw. des zeitlichen Verlaufs des Blutdrucks, wobei lediglich eine geringe Vorspannkraft im Vergleich zu den im Stand der Technik bekannten Messvorrichtungen erforderlich ist. Dadurch wird ein für den Patienten unangenehmes Druckgefühl und Unbehagen vermieden. Die erfindungsgemäße Vorrichtung zur Blutdruckmessung eignet sich daher auch in besonderer Weise zur Langzeitüberwachung eines Patienten.

Im Rahmen der Erfindung wird es bevorzugt, dass der Biegesensor ein piezoelektrischer Sensor ist. Derartige Sensoren sind klein und zeichnen sich durch eine hohe Empfindlichkeit aus.

Vorzugsweise kann der Biegesensor als Bimorph-Sensoranordnung mit zwei um die neutrale Faser angeordneten Einzelsensoren ausgebildet sein. Die Einzelsensoren besitzen antiparallele Polarität und sind symmetrisch um die neutrale Faser angeordnet. Bei einer Biegung der an dem Träger angeordneten Bimorph-Sensor-anordnung in eine Richtung wird einer der Einzelsensoren gedehnt, während der andere Einzelsensor gleichermaßen gestaucht wird. Bei einer derartigen entgegengesetzten Belastung der beiden Einzelsensoren addieren sich deren Signale.

Es liegt auch im Rahmen der Erfindung, dass der Biegesensor der erfindungsgemäßen Vorrichtung als Multimorph-Biegesensor ausgebildet ist und mehrere Paare von Einzelsensoren mit abwechselnd antiparalleler Polarität aufweist. Durch das Vorsehen mehrerer Paare von Einzelsensoren kann die Empfindlichkeit erhöht werden.

Erfindungsgemäß ist vorgesehen, dass ein Schenkel oder beide Schenkel gelenkig an dem Träger angeordnet sind. Die gelenkige Anbringung ermöglicht eine Anpassung an unterschiedlich große Körperteile, zum Beispiel an unterschiedlich große Finger. Ein Schenkel oder beide Schenkel des Trägers besitzen eine Auflage.

Vorzugsweise ist die Auflage so ausgestaltet, dass eine definierte Ankopplung an ein darunter liegendes Gefäß wie eine Arterie oder Vene stattfindet. Vorzugsweise kann die Auflage eine schmale, linienförmige Erhebung aufweisen, die senkrecht auf das Gefäß aufgelegt werden kann. Bei einer am Finger tragbaren Vorrichtung ist die Auflage vorzugsweise kreissegmentförmig ausgebildet.

Die erfindungsgemäße Vorrichtung zur Blutdruckmessung kann ein Klemmelement aufweisen, um einen Schenkel mit einer definierten Kraft zu beaufschlagen. Selbstverständlich können auch beide Schenkel ein derartiges Klemmelement aufweisen. Die erfindungsgemäße Vorrichtung umfasst somit den an dem Träger angeordneten Biegesensor, die beiden abgewinkelten Schenkel, die Auswerteeinheit und optional wenigstens ein Klemmelement.

Eine pulsdruckbedingte Pulsation eines Volumens, insbesondere eines Körperteils eines Patienten, an dem eine Blutdruckmessung erfolgen soll, wird erfindungsgemäß durch eine U-Form des Trägers in eine Biegung gewandelt.

Dabei wird über das Klemmelement der pulsierende Bereich des Volumens zwischen die Schenkel des U-förmigen Trägers mit einer vorzugsweise definierten Vorspannkraft geklemmt. Auf diese Weise kann die pulsdruckbedingte Pulsation des Körperteils oder eines Abschnitts des Körperteils, insbesondere eines Fingers, durch die U-Form des Trägers in eine Biegung gewandelt werden. Diese Biegung wird von dem Biegesensor erfasst. Der Biegesensor befindet sich vorzugsweise an der Stelle, an der die Biegung maximal ist.

Über das Klemmelement wird eine vorzugsweise konstante Anpresskraft auf das Körperteil aufgebracht. Bevorzugt überdeckt die Anpressfläche des Klemmelements diejenigen Bereiche, unter denen die pulsierenden Gefäße verlaufen. Die Einspannfläche (= Anpressfläche) des Klemmelements ist bevorzugt derart ausgestaltet, dass sich die Kraft gleichmäßig über die Einspannfläche verteilen kann. Dies kann beispielsweise durch ein hautfreundliches Polster aus einem flexiblen Material wie Gummi oder Schaum oder mittels eines Luftkissens erreicht werden.

Mit einer derartigen erfindungsgemäßen Vorrichtung zur Blutdruckmessung kann eine durch eine arterielle und/oder venöse Pulsation verursachte schwache Auslenkung eines zumindest teilweise darin eingespannten oder davon umfassten Körperteils bzw. dessen Oberfläche in eine Biegung am Ort des Biegesensors und damit in ein elektrisches Signal gewandelt werden.

Insbesondere kann mit Hilfe einer solchen Anordnung die arterielle und/oder venöse Pulsation im Finger, die durch die oberflächennahen Blutgefäße verursacht wird, welche sich als pulsierende lokale Auslenkung der Oberfläche- bzw. des Volumens bestimmter Stellen am Finger äußert, mechanisch auf den Biegesensor übertragen und durch den Biegesensor in ein elektrisches Signal gewandelt werden, wodurch der Pulsdruckverlauf von einer Messelektronik in der Auswerteeinheit erfasst werden kann.

Das erfasste elektrische Sensorsignal weist ein hohes Signal/Rauschverhältnis auf. Aufgrund der hohen Signalgüte des elektrischen Sensorsignals kann der Pulsdruckverlauf mit allen systolischen und diastolischen Komponenten detailgetreu wiedergegeben werden.

Durch die hohe Empfindlichkeit der erfindungsgemäßen Vorrichtung ist es ohne Weiteres möglich, die Pulssignale selbst bei Verwendung eines bequemen Polsters und einer sehr niedrigen und damit maximal komfortablen Befestigungskraft mit hoher Genauigkeit zu detektieren. Somit löst eine durch eine Pulsation des Blutdrucks verursachte Auslenkung eines oder beider Schenkel eine von dem Biegesensor erfasste Biegung des Trägers aus, die von der Auswerteeinheit ausgewertet werden kann. Vorzugsweise kann durch die Auswerteeinheit der zeitliche Verlauf des Blutdrucks ermittelt werden.

Die Ermittlung von Vitaldaten (z. B. Blutdruckwerte, Puls) kann durch Analyse der Charakteristika (z. B. Extremwerte und deren zeitliche Abstände) des Pulsdrucckurvenverlaufs erfolgen.

Die Ermittlung von Vitaldaten kann auch über künstliche Intelligenz bzw. über maschinelles Lernen erfolgen, deren Netzwerke zuvor mit einer umfassenden Patientendatenbank trainiert wurden. Die Trainingsdatenbank kann dabei neben den Messkurven auch beliebige andere medizinische Daten bzw. Diagnosen der Patienten enthalten.

Im einfachsten Fall ist die von dem Klemmelement ausgeübte Kraft konstant. Es sind jedoch auch Ausführungen möglich, bei denen die von dem Klemmelement ausgeübte Kraft einstellbar ist. Bei der erfindungsgemäßen Vorrichtung kann sich ein Klemmelement einerseits an dem Träger und andererseits an einem Schenkel abstützen.

Wie bereits erwähnt, ist der Träger und/oder der die Schenkel aufweisende Träger der erfindungsgemäßen Vorrichtung U-förmig ausgebildet.

Nachfolgend werden die wesentlichen Komponenten der Vorrichtung zur Blutdruckmessung beschrieben.

Im Hinblick auf das Sensorelement sieht die bevorzugte Variante wenigstens einen Biegesensor vor, vorzugsweise eine Bimorph-Biegesensoranordnung mit antiparalleler Polarität. Alternativ können jedoch auch Multimorph-Biegesensoren verwendet werden, die aus mehreren Sensorpaaren (in Form von Folien oder Balken) mit abwechselnd antiparalleler Polarität zusammengesetzt sind. Die Bimorph-Biegesensoranordnung ist besonders bevorzugt eine piezoelektrische Bimorph-Biegesensoranordnung, bei der die Sensoren piezoelektrische Sensoren sind. Ein solches Sensorelement reagiert sehr empfindlich auf Biegung. Eine Bimorph-Biegesensoranordnung besteht im Prinzip aus zwei Sensorschichten, die symmetrisch um die neutrale Faser (Balkentheorie) angeordnet sind. Bei Biegung dieser Anordnung in eine Richtung, wird eine der sensorisch aktiven Biegesensorschichten gedehnt, während die andere gleichermaßen gestaucht wird. Bei Biegung in die andere Richtung verhält es sich genau umgekehrt. Durch eine antiparallele Polarität der zwei Sensorschichten addieren sich die Signale dieser gegenläufigen Belastungen konstruktiv (da gleiches Vorzeichen) also betragsmäßig und vergrößern das Gesamtsignal. Gleichläufige Effekte hingegen (z. B. störende Temperatureffekte, pyroelektrische Effekte) werden weitgehend ausgelöscht und damit kompensiert. Neben einem Biegesensor kann das Sensorelement zusätzlich auch andere Sensorelemente wie z. B. einen optischen Sensor enthalten.

Der Träger, an dem der Biegesensor angeordnet ist, weist die beiden Schenkel auf. Der Träger ist somit U-förmig ausgebildet und weist wenigstens einen Biegesensor, vorzugsweise ein Biegesensorelement auf. Werden die Schenkel des U-förmigen Trägers gegeneinander ausgelenkt, resultiert diese Auslenkung in einer Biegung am Ort des Sensorelements.

Durch Einspannung eines pulsierenden Objektvolumens, beispielsweise eines Fingers, zwischen die Schenkel, bewegen sich die Schenkel gegeneinander senkrecht zur eingespannten Oberfläche. Durch den durch die Schenkel gegenüber dem Träger gebildeten Hebel können bei entsprechender Auslegung selbst kleinste Auslenkungen in eine Biegung am Ort des Sensors übertragen werden, so dass diese durch den Biegesensor detektiert werden. Der U-förmige Träger kann auch als Ring, ausgebildet sein, der von einem Patienten am Finger oder am Arm getragen werden kann.

Bei der erfindungsgemäßen Vorrichtung zur Blutdruckmessung ist der Biegesensor über eine flexible bzw. elastische Einheit so verbunden, dass die Vorrichtung mit einer definierten Befestigungskraft am pulsierenden Körperteil, beispielsweise an einem Finger, angelegt und festgeklemmt werden kann. Die pulsierenden Gefäße des Körperteils befinden sich unter einer Auflage des Schenkels oder einer Auflage des Klemmelements des Schenkels bzw. berühren die Auflage des Klemmelements.

Eine Flexibilität kann dadurch erreicht werden, dass das Klemmelement ein elastisches Element, vorzugsweise ein Federelement, aufweist. Vorzugsweise lässt sich das Klemmelement durch Krafteinwirkung, beispielsweise über einen Hebelmechanismus, öffnen. Besonders bevorzugt kann für eine höhere Flexibilität des Klemmelements zumindest ein Gelenk mit einem federnden Rückstellmechanismus vorgesehen sein. Der federnde Rückstellmechanismus kann auch einen elektromechanischen, pneumatischen, hydraulischen oder piezoelektrischen Aktuator umfassen. Bei einem pneumatischen Aktuator können beispielsweise eine manuelle Pumpe, ein Mehrwegeventil und ein Manometer vorgesehen sein, um eine definierte, vorzugsweise konstante Kraft des flexiblen Klemmelements zu realisieren. Statt einer manuellen Pumpe kann auch eine elektrische Pumpe mit einem Regelkreis vorgesehen sein.

Bevorzugt lässt sich die Federkraft eines solchen federnden Rückstellmechanismus' manuell oder automatisch einstellen. Manuell kann dies beispielsweise über einen Drehmomentschlüssel erfolgen. Besonders bevorzugt ist die Federkraft eines solchen federnden Rückstellmechanismus' unabhängig oder zumindest weitgehend unabhängig von der Auslenkung, zumindest innerhalb eines bestimmten Toleranzbereichs. Dementsprechend ist die Kraft des Klemmelements weitestgehend konstant.

Eine Flexibilität kann durch eine Feder oder ein elastisches Material, welches einen oder beide Klemmschenkel mit dem Träger verbindet, realisiert werden. Die Auflage bzw. die Auflagen des bzw. der Schenkel sind so ausgestaltet, dass diese die Stelle des Körperteils, an der der Blutdruck gemessen werden soll, reproduzierbar einspannen können. Dazu wird möglichst diejenige Stelle des Körperteils des Patienten ausgewählt, an der die Auslenkung maximal ist. Die Auflage kann so über der Arterie oder Vene platziert werden, dass sie die Arterie oder Vene kreuzt. Die Auflage bildet einen definierten Krafteinleitungspunkt. Die Auslenkung der Auflage durch die Pulsation der Arterie oder Vene wird auf den Biegesensor übertragen und kann von diesem erfasst werden.

Die Auflage kann sich bevorzugt an die Oberfläche der pulsierenden Körperstelle anpassen. Dazu kann Auflage des Klemmelements zumindest teilweise gepolstert sein. Bevorzugt besteht die Auflage bzw. das Polster aus hautfreundlichem Material. In einer besonders vorteilhaften Ausgestaltung ist der flexible Teil der Vorrichtung zur Blutdruckmessung wie ein Ring, z. B. ein Fingerring oder Armreif, ausgestaltet.

Die Kraft, mit der das Klemmelement am jeweiligen Körperteil anliegt, kann einstellbar ausgestaltet sein. Dies kann beispielsweise über mindestens eine verstellbare Feder erfolgen. In einer weiteren vorteilhaften Ausgestaltung befindet sich in der Vorrichtung auch eine Anzeige für die Spannkraft. Eine solche Anzeige, ist beispielsweise mit einer Zugfeder realisierbar, die mit einer mechanischen Übersetzung (z. B. Hebel, Flaschenzug, oder Zahnrad) eine kleine Kraft in einen großen Ausschlag eines Kraftzeigers umformt.

Die Vorrichtung kann eine Einstellbarkeit der Spannkraft auf wenige Bruchteile eines Newton aufweisen. Die Einstellbarkeit kann beispielsweise in Stufen von 1/10 Newton erfolgen. Sie kann jedoch auch in Stufen von bis zu 1/100 Newton erfolgen. Dadurch lässt sich die Vorrichtung zur Blutdruckmessung mit einer sehr kleinen und gleichzeitig definierten Spannkraft anbringen. Dies ist vorteilhaft für die Ermittlung von absoluten Blutdruckwerten.

In einer anderen vorteilhaften Ausgestaltung reguliert sich eine voreingestellte, durch eine Feder erzeugte Spannkraft beim Anbringen am Finger oder an einem anderen Körperteil automatisch. Dies kann beispielsweise elektronisch durch einen Kraftmessdose in Kombination mit einem Aktuator erfolgen.

In einer besonders bevorzugten Ausgestaltung wird eine möglichst lange und möglichst stark vorgespannte Druckfeder verwendet, deren Kraftänderung in einem begrenzten Einstellbereich nur einen Bruchteil des Einstellweges bezogen auf die Federgesamtlänge beträgt.

Daneben sind weitere Ausführungen der erfindungsgemäßen Vorrichtung zur Blutdruckmessung möglich. Die Vorrichtung kann durch Miniaturisierung auch ringförmig ausgestaltet sein. Der Ring wird bevorzugt über der Fingerkuppe vor dem ersten Gelenk getragen. Es ist aber auch möglich, den Ring an jeder beliebigen Stelle des Fingers zu tragen. Ein solcher Ring kann auch am Arm oder am Bein oder sogar am Hals getragen werden, da die für eine Pulsdrucksensitivität nötige Spannkraft sehr gering ist. Der Ring kann somit als Ringschmuck oder Armschmuck oder Halsschmuck ausgebildet sein. In ähnlicher Weise kann der Ring auch als Bestandteil einer Uhr oder einer Smartwatch ausgebildet sein.

In einer besonders vorteilhaften Ausgestaltung umfasst die Vorrichtung zur Blutdruckmessung ein zumindest teilweise elastisches Element, vorzugsweise ein teilweise linearelastisches Element, z. B. eine Spiralfeder, die den Finger zumindest teilweise umgibt. Im Folgenden wird dieser Teil als Ringelement bezeichnet. Durch die Einstellung einer definierten Spannkraft ist nur eine einmalige Kalibrierung der erfindungsgemäßen Vorrichtung notwendig. Dadurch kann die Vorrichtung im kalibrierten Zustand ausgeliefert werden.

Durch die Verwendung mehrerer Vorrichtungen an verschiedenen Körperregionen können Aussagen über z. B. bestimmte Gefäßkrankheiten in diesen Körperregionen getroffen werden. Zudem können mit mindestens zwei solcher Ringe an verschiedenen Positionen (z. B. am Arm oder Finger) ohne weiteres Aussagen über die Pulswellengeschwindigkeit aus der Phasenverschiebung zwischen den Signalen bestimmt werden.

Die Auswerteeinheit umfasst eine Messelektronik, die einen Verstärker, Signalkonditionierungselektronik, einen A/D-Wandler und eine Funk- und/oder Kabelschnittstelle enthalten. Zudem kann sie noch eine Anzeige und verschiedene Steuerelemente, z. B. Auswahlknöpfe, enthalten. Idealerweise befinden sich die Signalkonditionierung, der AD-Wandler und das Funkübertragungsmodul der Messelektronik am oder im Träger. Dieses überträgt das digitalisierte Signal z. B. über Bluetooth oder ein anderes Funkübertragungsverfahren zu einem Überwachungsgateway oder zu einem mobilen Anzeigegerät. Dies kann auch ein Smartphone oder eine Smartwatch sein. Der Träger kann auch als Teil einer Smartwatch ausgebildet sein, die alle zur Erfassung und Verarbeitung der Sensorsignale erforderlichen Komponenten aufweist, insbesondere dem Biegesensor zugeordnete Mess- und Elektronikkomponenten. Diese Komponenten können auch in Form von miniaturisierten und integrierten Schaltungen wie FPGAs vorliegen. Derartige Schaltungen können kostengünstig hergestellt und einfach in eine Smartwatch oder ähnliche System integriert werden.

Die Messelektronik kann zumindest teilweise jedoch auch über ein Kabel mit dem Biegesensor verbunden und in einem Gehäuse an einer anderen Position am Körper des Patienten, z. B. am Armgelenk, platziert sein. Dieses Gehäuse kann auch eine Anzeige enthalten.

Mit der erfindungsgemäßen Vorrichtung hat sich überraschenderweise gezeigt, dass der Pulsdruckverlauf mit einer außerordentlich hohen Signalgüte erfasst werden kann. Dadurch können selbst kleinste bisher nur invasiv messbare Schwankungen im Pulsdruckverlauf nichtinvasiv erfasst werden. Mit der erfindungsgemäßen Vorrichtung lassen sich somit mehr Zusammenhänge und damit auch Krankheitsbilder erschließen als dies mit bisherigen nichtinvasiven Verfahren möglich war.

Die Auswertung des Verlaufs erfolgt über analytische Auswertung der Kurvencharakteristika wie Peaks, z. B. Maxima, Minima, Notch, Peakformen, relative Peakpositionen, relative Peakamplituden, Pulsfrequenz, Flächen unter den Peaks, Flächen unter systolischen und diastolischen Kurvenabschnitten, und Verknüpfungen zwischen den Charakteristika untereinander.

Die erfindungsgemäße Vorrichtung ermöglicht die Erfassung von Vitaldaten, insbesondere Puls und Pulsdruckverlauf an nahezu jeder beliebigen Stelle des Körpers, selbst durch ein weiches Polster hindurch, indem sie so an der entsprechenden Körperstelle angebracht wird, dass sich zumindest ein oberflächennahes Blutgefäß unter der Kontaktfläche befindet.

Aufgrund der hohen Signalgüte der Pulsdruckverläufe kann selbst die Pulswellengeschwindigkeit über den Dicrotic Notch theoretisch ermittelt werden. Hierzu muss lediglich die Geometrie der untersuchten Körperteile vermessen werden und in die Berechnung mit einbezogen werden. Die Laufzeit bis zur reflektierten Pulswelle wird als Dicrotic Notch bezeichnet. Dadurch ermöglicht die erfindungsgemäße Vorrichtung die Anwendung als tragbares Überwachungsgerät zur Messung biomedizinischer Daten für medizinische Zwecke.

Daneben betrifft die Erfindung ein Verfahren zur Blutdruckmessung, mit den folgenden Schritten: Klemmen eines Körperteils zwischen zwei Schenkel einer Vorrichtung zur Blutdruckmessung, wobei die beiden Schenkel an entgegengesetzten Seiten eines Trägers, abgewinkelt zu dem Träger angeordnet sind, wobei an dem Träger ein Biegesensor angeordnet ist, der zum Erfassen einer Biegung des Trägers ausgebildet ist, und Ermitteln des Blutdruckwerts durch eine Auswerteeinheit anhand von Sensorsignalen des Biegesensors.

Weitere Vorteile und Einzelheiten der Erfindung werden nachfolgend anhand von Ausführungsbeispielen unter Bezugnahme auf die Zeichnungen erläutert. Die Zeichnungen sind schematische Darstellungen und zeigen:
- Fig. 1: ein Ausführungsbeispiel einer erfindungsgemäßen Vorrichtung zur Blutdruckmessung, die an einem Finger eines Patienten angebracht ist;
- Fig. 2: eine Draufsicht der in Fig. 1 gezeigten Vorrichtung;
- Fig. 3: ein Detail der in Fig. 1 gezeigten Vorrichtung;
- Fig. 4: ein weiteres Detail der in Fig. 1 gezeigten Vorrichtung;
- Fig. 5: ein weiteres Ausführungsbeispiel einer erfindungsgemäßen Vorrichtung zur Blutdruckmessung im gespannten Zustand;
- Fig. 6: die in Fig. 5 gezeigte Vorrichtung im entspannten Zustand;
- Fig. 7: ein weiteres Ausführungsbeispiel einer erfindungsgemäßen Vorrichtung;
- Fig. 8: ein Ausführungsbeispiel einer an einem Armband angebrachten Vorrichtung zur Blutdruckmessung;
- Fig. 9: ein weiteres Ausführungsbeispiel einer an einem Armband angebrachten Vorrichtung zur Blutdruckmessung;
- Fig. 10: ein ähnliches Ausführungsbeispiel einer Vorrichtung zur Blutdruckmessung wie Fig. 9;
- Fig. 11: Beispiele für die in den Fig. 8 bis 10 gezeigten Vorrichtungen während der Blutdruckmessung;
- Fig. 12: ein weiteres Ausführungsbeispiel einer Vorrichtung zur Blutdruckmessung;
- Fig. 13: eine Ansicht von unten einer Vorrichtung zur Blutdruckmessung;
- Fig. 14: eine geschnittene Ansicht der Vorrichtung von Fig. 13; und
- Fig. 15: eine Draufsicht auf die Vorrichtung von Fig. 13.

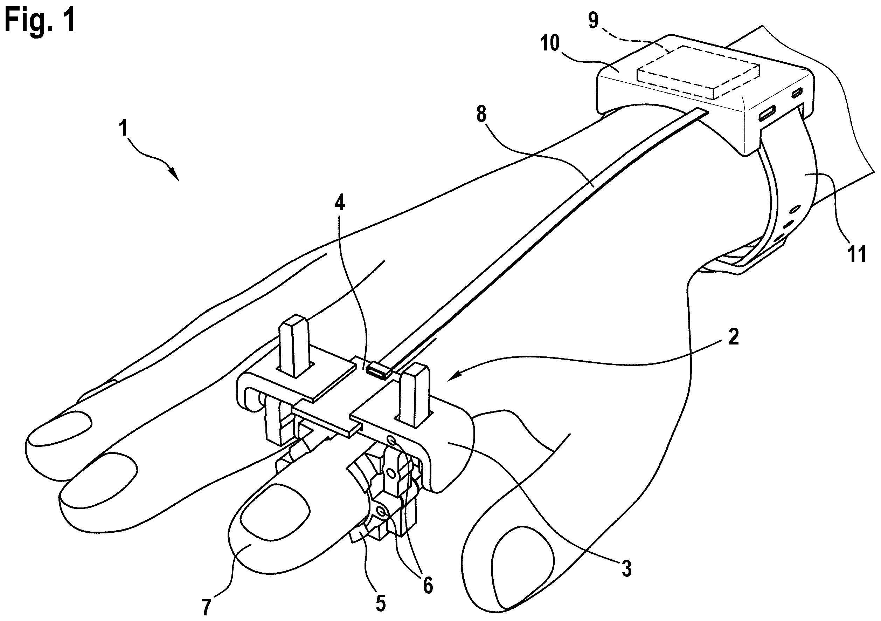

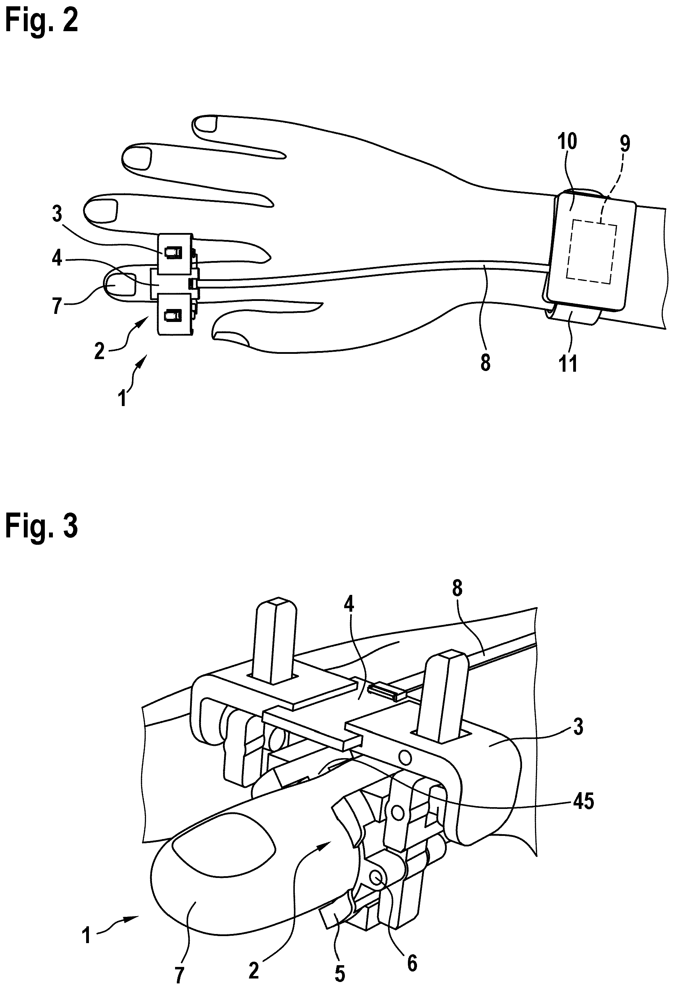

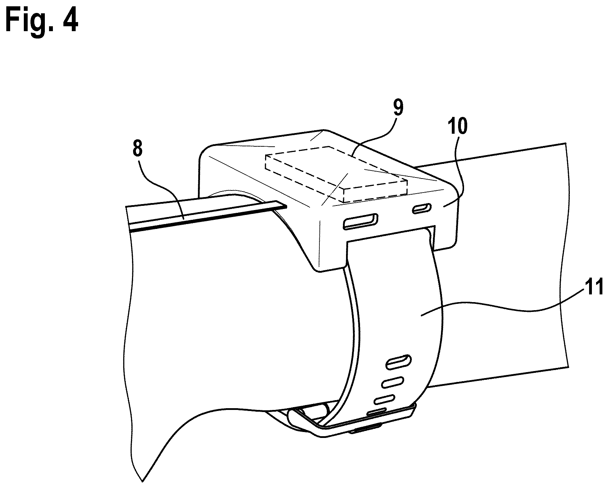

Die Fig. 1 bis 4 zeigen eine Vorrichtung zur Blutdruckmessung, wobei Fig. 1 die an einem Finger eines Patienten angebrachte Vorrichtung zeigt, Fig. 2 ist eine Draufsicht, Fig. 3 und Fig. 4 zeigen jeweils ein Detail der Vorrichtung.

Die Vorrichtung 1 umfasst einen zweiteilig ausgebildeten Träger 2, der zwei Schenkel 3 umfasst. Zwischen den beiden symmetrischen Schenkeln 3 ist ein Biegesensor 4 gehalten. Der durch die Schenkel 3 und den Biegesensor 4 gebildete Träger 2 ist U-förmig ausgebildet. Der Biegesensor 4 ist ein piezoelektrischer Sensor, der als bimorpher Sensor ausgebildet ist und zwei Einzelsensoren aufweist, die um die neutrale Faser des Biegesensors 4 angeordnet sind.

Jeder Schenkel 3 umfasst eine bogenförmige Auflage 5 mit einer schmalen linienförmigen zentralen Erhebung 45. Die Auflage 5 wird so angelegt, dass sie senkrecht zu den darunter liegenden Gefäßen des Patienten verläuft. Vorzugsweise ist die Auflage 5 in ein Polster integriert. Die Auflage 5 ist über zwei Gelenke 6 schwenkbar an der Unterseite des Schenkels 3 gelagert. In Fig. 1 erkennt man, dass die Form der bogenförmigen Auflage 5 an die Außenkontur des Zeigefingers 7 eines Patienten angepasst ist.

Der Biegesensor 4 ist über ein Kabel 8 mit einer Auswerteeinheit 9 verbunden. Die Auswerteeinheit 9 umfasst ein Gehäuse 10, das ein Armband 11 aufweist, sodass die Auswerteeinheit 9 wie eine Uhr am Handgelenk getragen werden kann.

Durch den Herzschlag des Patienten wird in den Blutgefäßen die arterielle und venöse Pulsation hervorgerufen, die auch am Zeigefinger eines Patienten messbar ist. Die Vorrichtung 1, insbesondere deren Auflage 5, umschließt den Zeigefinger 7 des Patienten mit einer bestimmten Anpresskraft oder Klemmkraft. Die Anpress- oder Klemmkraft wirkt in einem schmalen Bereich, der durch die Form der Auflage 5 festgelegt wird, auf die darunter verlaufenden arteriellen oder venösen Blutgefäße. Die arteriellen und/oder venöse Pulsation bewirkt eine minimale Bewegung der Auflage 5, so dass der leicht vorgespannte Biegesensor eine sich ändernde Biegung erfährt. Die dadurch hervorgerufene Biegung wird von dem Biegesensor 4 erfasst und in ein elektrisches Signal umgewandelt, das über das Kabel 8 an die Auswerteeinheit 9 übertragen wird. Anhand des elektrischen Signals ermittelt die Auswerteeinheit 9 den Blutdruck. Der Blutdruckwert kann unterschiedlich verwendet werden. Beispielsweise kann er in einem Speicher gespeichert werden, sodass er für eine spätere Auswertung zur Verfügung steht. Alternativ kann er auch auf einer Anzeige dargestellt werden. Es ist auch möglich, dass die Auswerteeinheit 9 Blutdruckwerte über eine drahtlose Kommunikationsverbindung an ein anderes Gerät überträgt.

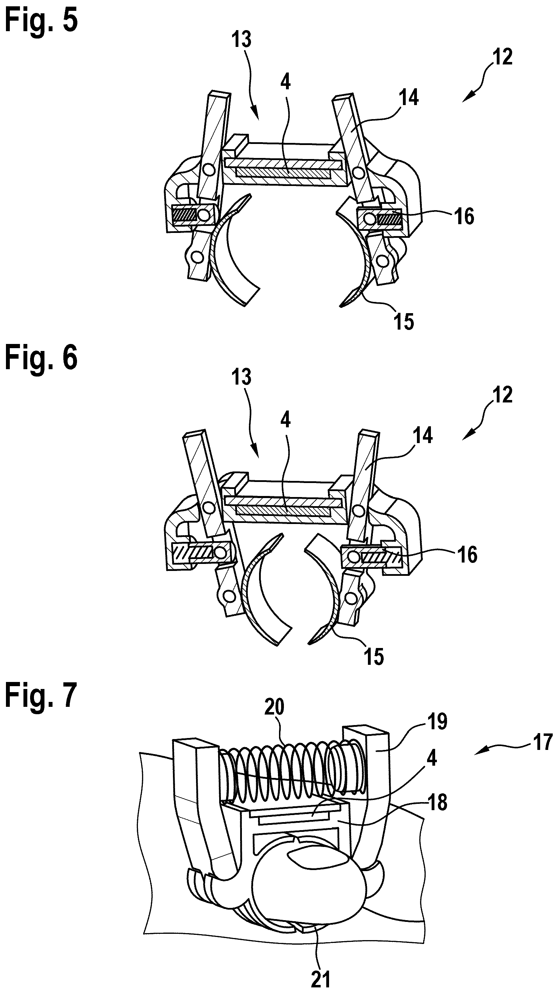

Die Fig. 5 und 6 sind geschnittene Darstellungen und zeigen eine Vorrichtung 12 zur Blutdruckmessung, wobei Fig. 5 die Vorrichtung im gespannten Zustand und Fig. 6 die Vorrichtung im entspannten Zustand zeigt. In Übereinstimmung mit dem ersten Ausführungsbeispiel umfasst die Vorrichtung 12 einen U-förmigen Träger 13 mit zwei symmetrischen Schenkeln 14, die gelenkig an dem Träger 13 befestigt sind. In dem Träger 13 ist der Biegesensor 4 aufgenommen. Jeder Schenkel 14 besitzt eine gebogene Auflage 15, die so geformt ist, dass beide Auflagen 15 einen Finger eines Patienten teilweise umschließen können. Die beiden Auflagen 15 sind zumindest näherungsweise als Kreissegmente ausgebildet. Die Auflagen 15 können ein Polster aufweisen, das zum Beispiel aus geschäumtem Kunststoff, aus Gummi oder aus einem Silikonmaterial bestehen kann. Das Polster besitzt eine schmale linienförmige Erhebung, die eine definierte punktuelle Auflagefläche bildet. Alternativ kann auch eine Auflage 15 unmittelbar eine schmale linienförmige Erhebung als Auflagefläche aufweisen. Diese schmalen linienförmigen Erhebungen werden möglichst senkrecht auf die darunter liegenden Blutgefäße eines Patienten gelegt. Dadurch ergeben sich an den Kreuzungen zwischen den schmalen linienförmigen Erhebungen und den Blutgefäßen zu definierten Ankopplungen. Bei herkömmlichen breiten Auflagen, die keine Erhebungen aufweisen, kann keine definierte Ankopplung entstehen. Das Polster kann auch ein Gel enthalten oder mit Luft gefüllt sein.

In den geschnittenen Ansichten von Fig. 5 und 6 erkennt man, dass beide Schenkel 14 jeweils ein Klemmelement 16 aufweisen, das als Federelement, genauer gesagt als Spiralfeder, ausgebildet ist. Das Klemmelement 16 stützt sich einerseits an dem U-förmigen Träger 13 und andererseits an dem Schenkel 14 ab. Wenn die Vorrichtung 12 an einem Finger angebracht ist, werden die Klemmelemente 16 komprimiert und üben eine definierte Anpresskraft auf den Finger aus. Dieser Zustand ist in Fig. 5 gezeigt. Andererseits, wenn die Vorrichtung 12 nicht zur Blutdruckmessung benutzt wird, sind die Klemmelemente 16 entspannt, sodass die Auflagen 15 der Schenkel 14 zueinander gedrückt werden.

Fig. 7 zeigt ein weiteres Beispiel einer Vorrichtung 17 zur Blutdruckmessung. Die Vorrichtung 17 umfasst einen Träger 18 mit dem Biegesensor 4 und zwei U-förmigen Schenkeln 19, die so angeordnet sind, dass sie sich bei der Blutdruckmessung oberhalb des Fingers des Patienten befinden. Zwischen den beiden Schenkeln 19 ist eine Spiraldruckfeder als Klemmelement 20 angeordnet. An dem entgegengesetzten Ende der Schenkel 19 befindet sich jeweils eine Auflage 21 für den Finger. Durch die Wirkung des Klemmelements 20 werden die Schenkel 19 auseinander und die beiden Auflagen 21 zueinander gedrückt, sodass die Auflagen 21 mit einer definierten Kraft gegen den Finger gedrückt werden.

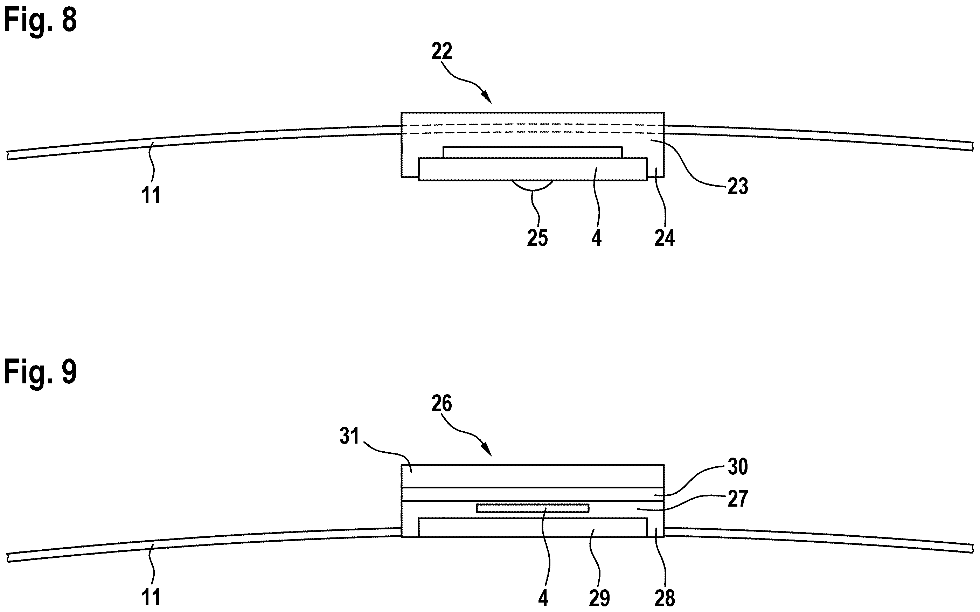

Fig. 8 zeigt ein Ausführungsbeispiel einer Vorrichtung 22, die an einem Armband 11 angebracht ist. Die Vorrichtung 22 umfasst einen U-förmigen Träger 23, der den Biegesensor 4 aufweist. In diesem Ausführungsbeispiel sind Schenkel 24 einstückig an dem Träger 23 angeformt. An derjenigen Seite der Vorrichtung 22, die zur Blutdruckmessung auf die Körperoberfläche direkt über einer Arterie eines Patienten aufgelegt werden kann, befindet sich auf dem Biegesensor 4 eine linienförmige Auflage 25, die in der geschnittenen Ansicht von Fig. 8 als Kreissegment bzw. Wölbung dargestellt ist. Die Auflage 25 dient zur gezielten und definierten Positionierung der Vorrichtung 25 oberhalb einer senkrecht dazu verlaufenden Arterie. Die Vorrichtung 22 kann vorzugsweise am Handgelenk getragen werden.

Fig. 9 zeigt ein ähnliches Ausführungsbeispiel wie Fig. 8. Die Vorrichtung 26 umfasst einen U-förmigen Träger 27 mit dem daran angeordneten Biegesensor 4. Der Träger 27 besitzt einstückig mit ihm ausgebildete abgewinkelte Schenkel 28, die an dem Armband 11 angebracht sind. Die Schenkel 28 schließen ein Polster 29 ein, das zur Blutdruckmessung auf der Körperoberfläche eines Patienten, beispielsweise im Bereich des Handgelenks, aufliegt. An der von dem U-förmigen Träger 27 nach außen weisenden Seite befindet sich ein weiteres Polster 30, auf dem eine Auswerteeinheit 31 befestigt ist. Bei der in Fig. 9 gezeigten Vorrichtung 26 ist keine Auflage dargestellt. Bei anderen Ausführungen kann sich eine Auflage an einer Position des Armbands 11 befinden, die sich im angelegten Zustand direkt über einer Arterie oder Vene befindet.

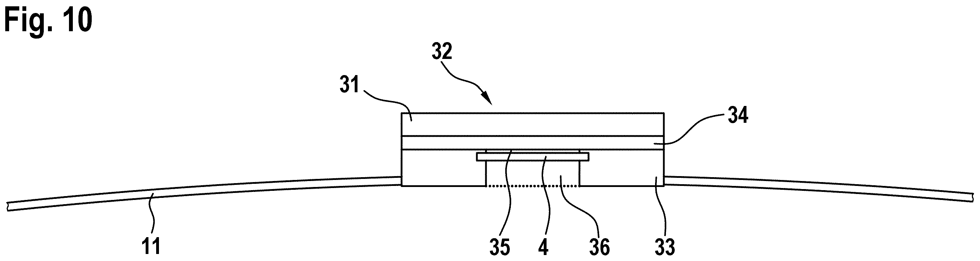

Fig. 10 zeigt eine ähnliche Vorrichtung 32 wie die in Fig. 9 gezeigte Vorrichtung 26. Die einen Träger bildenden Schenkel 33, zwischen denen der Biegesensor 4 angeordnet ist, sind allerdings separat ausgebildet. An der nach außen weisenden Seite der Schenkel 33 befindet sich ein Polster 34, auf dem die Auswerteeinheit 31 befestigt ist. An der Außenseite des Biegesensors 4 befindet sich ein Polster 35, an der Innenseite befindet sich ein Polster 36, das den Freiraum zwischen den Schenkeln 33 ausfüllt. Bei der in Fig. 10 gezeigten Vorrichtung 26 ist ebenfalls keine Auflage dargestellt. Bei anderen Ausführungen kann sich eine Auflage an einer Position des Armbands 11 befinden, die sich im angelegten Zustand direkt über einer Arterie oder Vene befindet.

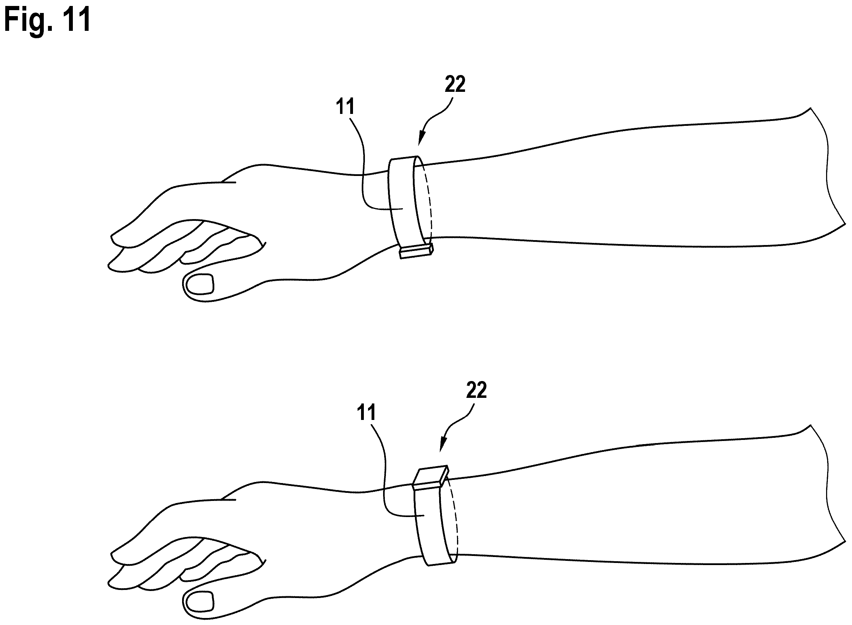

Fig. 11 zeigt Beispiele für die in den Fig. 8 bis 10 gezeigten Vorrichtungen während der Blutdruckmessung. Im oberen Teil von Fig. 11 ist die Vorrichtung 22 dargestellt, deren Armband sich am Handgelenk eines Patienten befindet. Die Vorrichtung 22 zur Blutdruckmessung liegt dabei an der Innenseite des Handgelenks des Patienten an, direkt über den arteriellen oder venösen Blutgefäßen. Anstelle der Vorrichtung 22 können auch die zuvor beschriebenen Vorrichtungen 26 und 32 an der gegenüberliegenden Position, d. h. an der Außenseite des Handgelenks, befestigt werden. Im unteren Teil von Fig. 11 befindet sich die Vorrichtung 22 an der Außenseite des Handgelenks und wird ähnlich wie eine Armbanduhr getragen.

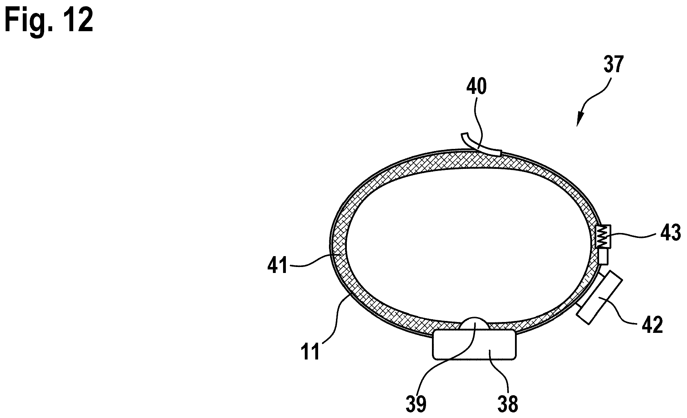

Fig. 12 zeigt ein Ausführungsbeispiel einer Vorrichtung 37, mit einem Armband 11, einem lediglich schematisch dargestellten Träger 38, der den Biegesensor (nicht gezeigt) aufweist. An der nach innen weisenden Seite besitzt der Träger 38 eine Auflage 39. Das Armband 11 besitzt ein Verschlusselement 40, zum Beispiel eine Schnalle oder einen Klettverschluss. An seiner Innenseite ist das Armband 11 mit einem Polster 41 versehen. Daneben umfasst das Armband 11 eine Vorrichtung 42 zum Einstellen einer Zugspannung, wobei die zu Spannung entweder manuell oder automatisch einstellbar ist. An dem Armband 11 ist auch eine Anzeige 43 zum Anzeigen der Zugspannung oder der Befestigungskraft vorhanden.

Es sind mehrere Abwandlungen der Vorrichtung 37 möglich. Beispielsweise kann die Auflage 39 auch an einer von dem Träger 38 entfernten Stelle angeordnet sein, beispielsweise gegenüberliegend von dem Träger 38, in der Nähe des Verschlusselements 40. Das Polster 41 an der Innenseite des Armband 11 ist optional und kann somit auch entfallen.

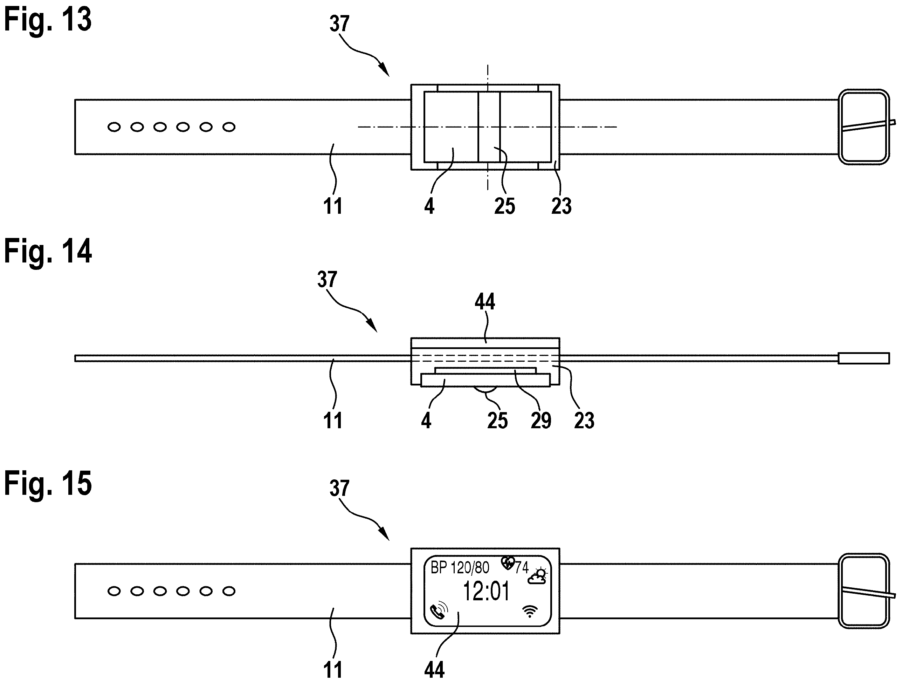

Die Fig. 13 bis 15 zeigen ein Ausführungsbeispiel der Vorrichtung 37, wobei Fig. 13 eine Ansicht von unten ist, Fig. 14 ist eine geschnittene Ansicht und Fig. 15 ist eine Draufsicht. Die Vorrichtung 37 umfasst das Armband 11, an dem sich der Träger 23 mit dem Biegesensor 4 befindet. An der Unterseite des Biegesensors 4 befindet sich eine schmale streifenförmige Auflage 25. In einem Freiraum zwischen den Schenkeln des Trägers 23 befindet sich ein Polster 29. Die Vorrichtung 37 ist als "Smartwatch" ausgebildet. Auf einer Anzeige 44 können neben Blutdruckwerten beispielsweise der Puls und andere Informationen - ähnlich wie bei einem Smartphone - dargestellt werden.

### Bezugszeichenliste

- 1: Vorrichtung
- 2: Träger
- 3: Schenkel
- 4: Biegesensor
- 5: Auflage
- 6: Gelenk
- 7: Zeigefinger
- 8: Kabel
- 9: Auswerteeinheit
- 10: Gehäuse
- 11: Armband
- 12: Vorrichtung
- 13: Träger
- 14: Schenkel
- 15: Auflage
- 16: Klemmelement
- 17: Vorrichtung
- 18: Träger
- 19: Schenkel
- 20: Klemmelement
- 21: Auflage
- 22: Vorrichtung
- 23: Träger
- 24: Schenkel
- 25: Auflage
- 26: Vorrichtung
- 27: Träger
- 28: Schenkel
- 29: Polster
- 30: Polster
- 31: Auswerteeinheit
- 32: Vorrichtung
- 33: Schenkel
- 34: Polster
- 35: Polster
- 36: Polster
- 37: Vorrichtung
- 38: Träger
- 39: Auflage
- 40: Verschlusselement
- 41: Polster
- 42: Vorrichtung
- 43: Anzeige
- 44: Anzeige
- 45: Erhebung

## Patentansprüche

1. Vorrichtung (1, 12, 17) zur Blutdruckmessung an einem Blutgefäße aufweisenden Körperteil, umfassend:
- einen Träger (2, 13, 18),
- einen an dem Träger (2, 13, 18) angeordneten Biegesensor (4), der zum Erfassen einer Biegung des Trägers (2, 13, 18) ausgebildet ist,
- eine Auswerteeinheit (9), die zum Ermitteln eines Blutdruckwerts anhand von Sensorsignalen des Biegesensors (4) ausgebildet ist,
wobei der Träger (2, 13, 18), in dem der Biegesensor (4) aufgenommen ist, U-förmig ausgebildet ist und zwei Schenkel (3, 14, 19) aufweist, die an entgegengesetzten Seiten des Trägers (2, 13, 18) abgewinkelt zu dem Träger (2, 13, 18) angeordnet sind, wobei der Biegesensor (4) zwischen den beiden Schenkeln (3, 14, 19) gehalten ist, wobei die Schenkel (3, 14, 19) jeweils eine Auflage (5, 15, 21) besitzen, wobei ein Schenkel (3, 14, 19) oder beide Schenkel (3, 14, 19) gelenkig an dem Träger (2, 13, 18) angeordnet sind,
wobei die Vorrichtung (1, 12, 17) so ausgebildet ist, dass eine durch eine arterielle und/oder venöse Pulsation der Blutgefäße des Körperteils bewirkte Bewegung der Auflage (5, 15, 21) über die Schenkel (3, 14, 19) des U-förmigen Trägers (2, 13, 18) eine Biegung des Biegesensors (4) hervorruft, die in das Sensorsignal umgewandelt wird.

2. Vorrichtung nach Anspruch 1, wobei der Biegesensor (4) ein piezoelektrischer Sensor ist.

3. Vorrichtung nach Anspruch 1 oder 2, wobei der Biegesensor (4) als Bimorph-Sensor-Anordnung mit zwei um die neutrale Faser angeordneten Einzelsensoren ausgebildet ist.

4. Vorrichtung nach einem der vorangehenden Ansprüche, wobei der Biegesensor (4) als Multimorph-Biegesensor ausgebildet ist und mehrere Paare von Einzelsensoren mit abwechselnd antiparalleler Polarität aufweist.

5. Vorrichtung nach einem der vorangehenden Ansprüche, wobei die Schenkel (3) jeweils eine Auflage (5, 15, 21) besitzen, die kreissegmentförmig ist.

6. Vorrichtung nach einem der vorangehenden Ansprüche, wobei wenigstens ein Klemmelement (16, 20) vorgesehen ist, um einen Schenkel (14, 19) mit einer definierten Kraft zu beaufschlagen.

7. Vorrichtung nach Anspruch 6, wobei jedem Schenkel (14, 19) ein Klemmelement (16, 20) zugeordnet ist.

8. Vorrichtung nach Anspruch 6 oder 7, wobei das Klemmelement (16, 20) ein Federelement aufweist, durch das entweder ein Schenkel (14, 19) oder beide Schenkel (14, 19) mit einer Kraft beaufschlagbar ist oder sind.

9. Vorrichtung nach einem der Ansprüche 6 bis 8, wobei die von dem Klemmelement (16, 20) ausgeübte Kraft einstellbar ist.

10. Vorrichtung nach einem der Ansprüche 6 bis 8, wobei ein Klemmelement (16, 20) sich einerseits an dem Träger (13) und andererseits an einem Schenkel (14) abstützt.

11. Vorrichtung nach einem der vorangehenden Ansprüche, wobei die Auswerteeinheit (9) zum Ermitteln eines zeitlichen Verlaufs des Blutdrucks ausgebildet ist.

12. Verfahren zur Blutdruckmessung, mit den folgenden Schritten:
- Klemmen eines Körperteils zwischen zwei Schenkel (3, 14, 19) einer Vorrichtung (1, 12, 17) zur Blutdruckmessung nach einem der Ansprüche 1 bis 11, wobei die beiden Schenkel (3, 14, 19) an entgegengesetzten Seiten eines Trägers (2, 13, 18) abgewinkelt zu dem Träger (2, 13, 18) angeordnet sind, wobei ein Schenkel (3, 14, 19) oder beide Schenkel (3, 14, 19) gelenkig an dem Träger (2, 13, 18) angeordnet sind, wobei an dem Träger (2, 13, 18) ein Biegesensor (4) angeordnet ist, der zum Erfassen einer Biegung des Trägers (2, 13, 18) ausgebildet ist, und
- Ermitteln des Blutdruckwerts durch eine Auswerteeinheit (9) anhand von Sensorsignalen des Biegesensors (4).

## Claims

1. A device (1, 12, 17) for blood pressure measurement on a part of the body with blood vessels, comprising:
- a support (2, 13, 18),
- a bending sensor (4) arranged on the support (2, 13, 18), which is designed to detect a bend in the support (2, 13, 18),
- an evaluation unit (9) which is designed to determine a blood pressure value using sensor signals from the bending sensor (4),
wherein the support (2, 13, 18), in which the bending sensor (4) is accommodated, is U-shaped, and comprises two legs (3, 14, 19), which are arranged on opposite sides of the support (2, 13, 18) at an angle to the support (2, 13, 18), wherein the bending sensor (4) is held between the two legs (3, 14, 19), wherein the legs (3, 14, 19) each have a support (5, 15, 21), wherein one leg (3, 14, 19) or both legs (3, 14, 19) are arranged in an articulated manner on the support (2, 13, 18),
wherein the device (1, 12, 17) is designed such that a movement of the support (5, 15, 21), caused by an arterial and/or venous pulsation of the blood vessels of the body part, causes a bending of the bending sensor (4), via the legs (3, 14, 19) of the U-shaped support (2, 13, 18), wherein said bending is converted into the sensor signal.

2. Device according to claim 1, wherein the bending sensor (4) is a piezoelectric sensor.

3. Device according to claim 1 or 2, wherein the bending sensor (4) is designed as a bimorph sensor arrangement with two individual sensors arranged around the neutral fiber.

4. Device according to any one of the preceding claims, wherein the bending sensor (4) is designed as a multimorph bending sensor and has several pairs of individual sensors with alternating antiparallel polarity.

5. Device according to any one of the preceding claims, wherein the legs (3) each have a support (5, 15, 21) which is in the shape of a segment of a circle.

6. Device according to any one of the preceding claims, wherein at least one clamping element (16, 20) is provided in order to apply a defined force to a leg (14, 19).

7. Device according to claim 6, wherein a clamping element (16, 20) is assigned to each leg (14, 19).

8. Device according to claim 6 or 7, wherein the clamping element (16, 20) has a spring element by means of which either one leg (14, 19) or both legs (14, 19) can be subjected to a force.

9. Device according to any one of claims 6 to 8, wherein the force exerted by the clamping element (16, 20) is adjustable.

10. Device according to any one of claims 6 to 8, wherein a clamping element (16, 20) is supported on one side on the support (13) and on the other side on a leg (14).

11. Device according to one of the preceding claims, wherein the evaluation unit (9) is designed to determine a temporal course of the blood pressure.

12. Method for blood pressure measurement, with the following steps:
- clamping of a body part between two legs (3, 14, 19) of a device (1, 12, 17) for measuring blood pressure according to any one of claims 1 to 11, the two legs (3, 14, 19) being arranged on opposite sides of a support (2, 13, 18), angled with respect to the support (2, 13, 18), wherein one leg (3, 14, 19) or both legs (3, 14, 19) are arranged in an articulated manner on the support (2, 13, 18), a bending sensor (4) being arranged on the support (2, 13, 18), which is designed to detect a bending of the support (2, 13, 18), and
- determination of the blood pressure value by an evaluation unit (9) using sensor signals from the bending sensor (4).

## Revendications

1. Dispositif (1, 12, 17) de mesure de la pression artérielle sur une partie du corps présentant des vaisseaux sanguins, comprenant :
- un support (2, 13, 18),
- un capteur de flexion (4) disposé sur le support (2, 13, 18), qui est conçu pour détecter une flexion du support (2, 13, 18),
- une unité d'évaluation (9) conçue pour évaluer une valeur de pression artérielle sur la base de signaux de capteur émis par le capteur de flexion (4),
en ce que le support (2, 13, 18), dans lequel est logé le capteur de flexion (4), est en forme de U et comporte deux branches (3, 14, 19) qui sont disposées sur des côtés opposés du support (2, 13, 18) à un certain angle par rapport au support (2, 13, 18), en ce que le capteur de flexion (4) est maintenu entre les deux branches (3, 14, 19), en ce que les branches (3, 14, 19) comportent chacune un appui (5, 15, 21), en ce qu'une branche (3, 14, 19) ou les deux branches (3, 14, 19) sont disposées de manière articulée sur le support (2, 13, 18),
en ce que le dispositif (1, 12, 17) est conçu de telle sorte qu'un mouvement de l'appui (5, 15, 21) causé par une pulsation artérielle et/ou veineuse des vaisseaux sanguins de la partie du corps provoque une flexion du capteur de flexion (4) par l'intermédiaire des branches (3, 14, 19) du support (2, 13, 18) en forme de U, ladite flexion étant convertie en signal de capteur.

2. Dispositif selon la revendication 1, en ce que le capteur de flexion (4) est un capteur piézoélectrique.

3. Dispositif selon la revendication 1 ou 2, en ce que le capteur de flexion (4) est conçu comme un ensemble de capteurs bimorphes avec deux capteurs individuels disposés autour de la fibre neutre.

4. Dispositif selon l'une des revendications précédentes, en ce que le capteur de flexion (4) est conçu comme capteur de flexion multimorphe et comporte plusieurs paires de capteurs individuels avec une polarité antiparallèle alternée.

5. Dispositif selon l'une des revendications précédentes, en ce que les branches (3) ont chacune un appui (5, 15, 21) qui est en forme de segment de cercle.

6. Dispositif selon l'une des revendications précédentes, en ce qu'au moins un élément de serrage (16, 20) est prévu pour appliquer une force définie sur une branche (14, 19).

7. Dispositif selon la revendication 6, en ce qu'un élément de serrage (16, 20) est attribué à chaque branche (14, 19).

8. Dispositif selon la revendication 6 ou 7, en ce que l'élément de serrage (16, 20) comporte un élément à ressort qui permet d'appliquer une force soit sur une branche (14, 19) ou sur les deux branches (14, 19).

9. Dispositif selon l'une des revendications 6 à 8, en ce que la force exercée par l'élément de serrage (16, 20) est réglable.

10. Dispositif selon l'une des revendications 6 à 8, en ce qu'un élément de serrage (16, 20) repose d'une part sur le support (13) et d'autre part sur une branche (14).

11. Dispositif selon l'une des revendications précédentes, en ce que l'unité d'évaluation (9) est conçue pour déterminer une évolution temporelle de la pression artérielle.

12. Procédé de mesure de la pression artérielle, comprenant les étapes suivantes :
- le serrage d'une partie du corps entre deux branches (3, 4, 19) d'un dispositif (1, 12, 17) de mesure de pression artérielle selon l'une des revendications 1 à 11, en ce que les deux branches (3, 4, 19) sont disposées sur des côtés opposés d'un support (2, 13, 18), à un certain angle par rapport au support (2, 13, 18), en ce qu'une branche (3, 14, 19) ou les deux branches (3, 14, 19) sont disposées de manière articulée sur le support (2, 13, 18), en ce qu'un capteur de flexion (4) est disposé sur le support (2, 13, 18), ledit capteur de flexion étant conçu pour détecter une flexion du support (2, 13, 18), et
- la détermination de la valeur de la pression artérielle par une unité d'évaluation (9) sur la base de signaux de capteur émis par le capteur de flexion (4).
